# EUROPEAN PATENT SPECIFICATION

(11) **EP 2 397 565 B1**
(45) Date of publication and mention of the grant of the patent: **05.06.2013**
(21) Application number: 11170360.9
(22) Date of filing: 17.06.2011
(51) Int. Cl.: C12Q 1/68

(54) **Genetic risk assessment for shar-pei fever**
Genetische Risikobeurteilung für das Shar-Pei-Fieber
Évaluation du risque génétique dans la fièvre de shar-pei

(30) Priority: 18.06.2010 US 356230 P
(43) Date of publication of application: 21.12.2011
(73) Proprietor: Lindblad-Toh, Kerstin, 752 63 Uppsala (SE); Olsson, Mia, 741 92 Knivsta (SE); Tintle, Linda J. M., Wurtsboro NY 12790 (US)
(72) Inventor: Lindblad-Toh, Kerstin, 752 63 Uppsala (SE); Olsson, Mia, 741 92 Knivsta (SE); Tintle, Linda J. M., Wurtsboro, NY 12790 (US); Meadows, Jennifer Robyn Sylvia, 75220 Uppsala (SE)
(74) Representative: Johansson, Lars E.

(56) References cited:
- DATABASE EMBL [Online] 18 November 2009 (2009-11-18), "Sus scrofa GSS, clone WTSI_1005-541A6, end sequence WTSI_1005-541A6.F", XP000002658147, retrieved from EBI accession no. EMBL:HE324417 Database accession no. HE324417
- OLSSON MIA ET AL: "A Novel Unstable Duplication Upstream of HAS2 Predisposes to a Breed-Defining Skin Phenotype and a Periodic Fever Syndrome in Chinese Shar-Pei Dogs", PLOS GENETICS, vol. 7, no. 3, March 2011 (2011-03), XP000002658148,
- AKEY JOSHUA M ET AL: "Tracking footprints of artificial selection in the dog genome", PROCEEDINGS OF THE NATIONAL ACADEMY OF SCIENCES OF THE UNITED STATES OF AMERICA, vol. 107, no. 3, January 2010 (2010-01), pages 1160-1165, XP000002658149, ISSN: 0027-8424

## Description

### BACKGROUND OF THE INVENTION

### Field of the Invention

The present invention relates to the field of genetics and molecular biology. More specifically, the present invention relates to methods for risk assessment of the development of Familial Shar-Pei fever, an autoinflammatory disease common in the Shar-Pei dog breed.

### Related art

The Shar-Pei dog has been a companion animal within China for centuries commissioned to guard and hunt, and sometimes serving as a fighting dog. At the beginning of the communist era, dog ownership was associated with very high taxes and the breed was close to extinction when a few specimens were exported to the United States in the early 1970's. Shar-Pei descending from this handful of dogs have undergone strong selection for their wrinkled skin phenotype and are called the "meatmouth" type. An ancestral Shar-Pei referred to as the "bonemouth" type still occurs and it presents with a less accentuated skin condition.

The major constituent of the deposit in the thickened skin is hyaluronic acid (HA), a multifunctional, linear, high-molecular-mass glycosaminoglycan, widely spread throughout epithelial, connective and neural tissues⁷. The biological role of HA depends on its size, location and equilibrium between synthesis and degradation^{4,7}. Shar-Pei show two- to fivefold higher serum levels of HA compared to other breeds⁸, which has proposed the term *hyaluronanosis* also used for a comparable human condition⁹. The concentration of serum HA varies with age, between days, and with time of day as well as with sampling occasion**¹⁰**, suggesting that serum HA is an unstable measurement potentially influenced by random release of HA from other tissues. HA is synthesized by three enzymes, *Hyaluronic Acid Synthase (HAS) 1,2* and *3* **^{11,12}**, with *HAS2* being the rate limiting-enzyme**¹²**. *HAS2* is overexpressed in dermal fibroblasts of Shar-Pei compared to other canine breeds³ suggesting a regulatory mutation as causative for hyaluronanosis. HA is deposited in certain areas of the skin of Shar-Pei, often around the head and as "socks" around the hocks. Almost all Shar-Pei seem to be affected by hyaluronanosis, with the extent varying among individuals. However, most individuals show less skin folds and hyaluronanosis with age**⁹**.

Shar-Pei also suffer a strong predisposition for an autoinflammatory disease, Familial Shar-Pei Fever (FSF), that clinically resembles some hereditary periodic fever syndromes in humans, such as Familial Mediterranean Fever (FMF)². Both diseases are autoinflammatory, characterized by seemingly unprovoked episodes of inflammation. It presents in both FMF and FSF as short (12-72 hour) recurrent bouts of high fever, accompanied by localized inflammation usually involving the joints (especially the ankles in humans and the corresponding hock joint in dogs).

Patients with FMF and Shar-Pei with FSF seem free of symptoms between episodes that can occur as often as every few weeks. However, since acute phase reactants endure between episodes, a subclinical state and chronic autoinflammation might persist. The chronic state puts the patients at risk of developing reactive systemic amyloidosis and kidney or liver failure following the accumulation of acute phase proteins in the organs. In Shar-Pei, the episodes are typically more frequent during the first years of life and the frequency of Shar-Pei dogs with unexplained fever episodes was estimated to be 23% in the US in 1992².

Up to this point it has been unknown why Shar-Pei dogs are predisposed to FSF and there are currently no methods or genetic tests that can predict whether a Shar-Pei dog will develop FSF and the serious consequences of reactive amyloidosis and e.g. kidney failure. Such methods and tests would be of great value for breeders of Shar-Pei in their selection of breeder animals and would thus reduce the risk that future generations of Shar-Pei will develop FSF.

### DESCRIPTION OF THE INVENTION

The present invention provides methods for testing a Shar-Pei dog for its genetic predisposition to develop Familial Shar-Pei fever, the method comprising analyzing a nucleic acid sample obtained from said canine for a regulatory mutation in the Shar-Pei genome.

The present inventors have discovered a regulatory mutation, a 16.1 Kb duplication (SEQ ID NO:1), upstream of the Hyaluronic Acid Synthase 2 (HAS2) gene on chromosome 13 in Shar-Pei dogs. A high copy number of the duplication, present only in Shar-Pei, is associated with severe fever. Accordingly, genetic tests for the copy number of the duplication allow the assessment of the risk for a Shar-Pei dog to develop Shar-Pei fever. A high copy number (>10) of the duplication indicates a high risk for Shar-Pei fever, a low copy number (<5) a low risk, and a copy number between 5-10, an intermediary risk. As Shar-Pei fever is a serious disease, and its chronic state involves a high risk of developing systemic amyloidosis and kidney failure, a genetic test for the copy number of the duplication will be useful in Shar-Pei breeding.

One aspect of the present invention provides a method for testing a Shar-Pei dog for its genetic predisposition to develop Familial Shar-Pei fever which method comprises, or consists essentially of, obtaining sequence information from the region of nucleic acid located upstream of the Hyaluronic Acid Synthase 2 (HAS2) gene on the canine chromosome 13 to determine the number of duplications present in this region, wherein the number of duplications is indicative of the risk that the dog will develop Familial Shar-Pei fever.

Said region of nucleic acid located upstream of the Hyaluronic Acid Synthase 2 (HAS2) gene on the canine chromosome 13 comprises the nucleic acid sequence consisting of bp 22,937,592 to bp 24,414,650 (CanFam 2.0 Chr13: 22,937,592-24,414,650).

Obtaining sequence information can comprise sequencing a portion of the region of nucleic acid located on chromosome 13 CanFam 2.0 Chr13: 22,937,592-24,414,650 to determine the number of duplications present in the region. Preferably, the duplication can consist of the nucleic sequence SEQ ID NO:1 or sequence having more than 95 % identity to SEQ ID NO:1, such as more than 99 % identity to SEQ ID NO:1.

Obtaining sequence information can comprise using a nucleic acid probe capable of hybridizing to a part of the nucleic acid sequence SEQ ID NO:1, or a sequence complementary thereto, under stringent conditions.

Obtaining sequence information can comprise using a nucleic acid primer pair capable of amplifying a nucleic acid comprising a part of the duplication to determine the number of duplications present. The nucleic acid primers can be selected from SEQ ID NO:2, SEQ ID NO:3, SEQ ID NO:4, SEQ NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13., SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, and SEQ ID NO:23.

For those skilled in the art, there are many techniques available to analyze nucleic acids and to determine the copy number of a genetic element. Some of the techniques that can be used according to the present invention include; Real time PCR, Southern blot analysis, DNA sequencing and DNA array analysis.

In another aspect, the present invention provides an isolated nucleic acid probe comprising a sequence present in a duplication on the canine chromosome 13 corresponding to the nucleic acid sequence SEQ ID NO:1, and wherein the nucleic acid probe is capable of hybridizing to a part of the nucleic acid sequence SEQ ID NO:1, or a sequence complementary thereto, under stringent conditions.

In another aspect, the present invention provides an isolated nucleic acid primer pair comprising a first primer and a second primer, wherein each of the first and second primers comprises a sequence present in a duplication on the canine chromosome 13 corresponding to the nucleic acid sequence SEQ ID NO:1, and wherein the primer pair is capable of amplifying a nucleic acid containing at least a part of said duplication.

In another aspect, the present invention provides an isolated nucleic acid primer comprising a sequence present in a duplication on the canine chromosome 13 corresponding to the nucleic acid sequence SEQ ID NO:1.

In another aspect, the present invention provides an isolated nucleic acid primer pair comprising a first primer and a second primer, wherein each of the first and second primers comprises a sequence present in a duplication on the canine chromosome 13 corresponding to the nucleic acid sequence SEQ ID NO:1, and wherein the primer pair is capable of amplifying a nucleic acid containing a breakpoint of the duplication.

It is contemplated that any method or composition described herein can be implemented with respect to any other method or composition described herein. These, and other, embodiments of the invention will be better appreciated and understood when considered in conjunction with the following description and the accompanying drawings. It should be understood, however, that the following description, while indicating various embodiments of the invention and numerous specific details thereof, is given by way of illustration and not of limitation. Many substitutions, modifications, additions and/or rearrangements may be made within the scope of the invention without departing from the spirit thereof, and the invention includes all such substitutions, modifications, additions and/or rearrangements.

The use of the word "a" or "an" in the claims and/or the specification may mean "one", but it is also consistent with the meaning of "one or more", "at least one", and "one or more than one".

The phrase "one or more" found in the claims and/or the specification is defined as 1, 2, 3, 4, 5, 6, 7, 8, 9, 10, or more.

Throughout this application, the terms "about" and "approximately" indicate that a value includes the inherent variation of error for the device, the method being employed to determine the value, or the variation that exists among the study subjects. In one non-limiting embodiment the terms are defined within 10%, preferably within 5%, more preferably within 1%, and most preferably within 0.5%.

The use of the term "or" in the claims is used to mean "and/or" unless explicitly indicated to refer to alternatives only or the alternatives are mutually exclusive, although the disclosure supports a definition that refers to only alternatives and "and/or".

### BRIEF DESCRIPTION OF THE DRAWINGS

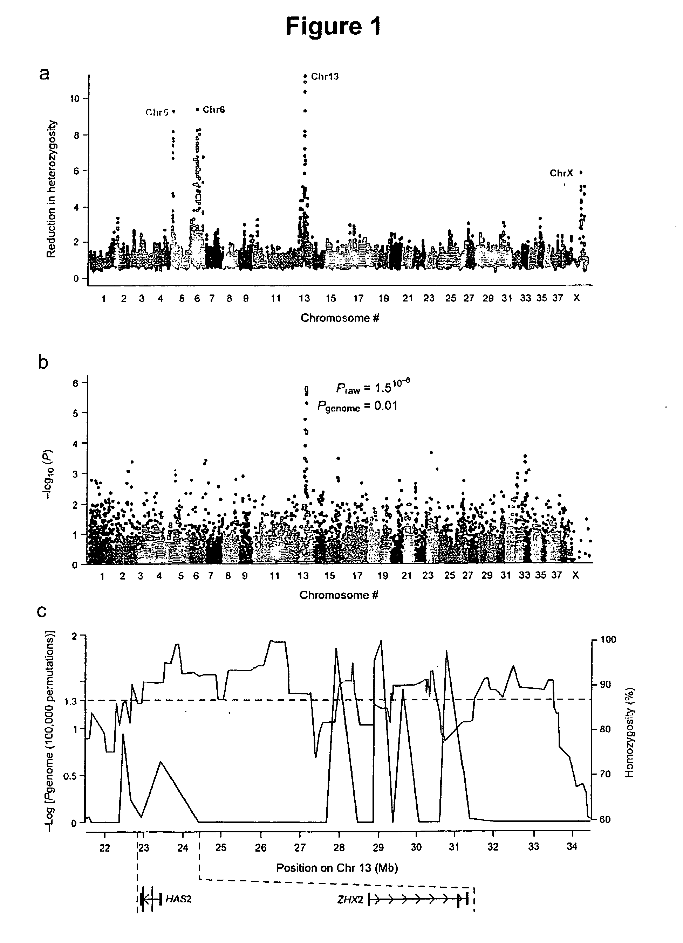

**Figure 1****. The breed-specific disease association and the strongest selective sweep signal in this breed are present in the same region on chromosome 13. (a)** A 10-fold reduction of heterozygosity was identified on chromosome 13 when comparing Shar-Pei (*n*=50) to 24 other canine breeds (*n*=230). 50,000 SNPs were used to screen the whole genome using a sliding window approach (see methods). **(b)** A case-control GWAs analysis identified five SNPs in a 2.7 Mb region on chromosome 13 (CanFam 2.0 Chr13 27,9- 30,7 Mb) significantly associated with Familial Shar-Pei Fever (FSF), after correcting for multiple testing using 100,000 permutations. The Shar-Pei dogs (*n* = 40) used in the study were strictly classified into groups of affected (*n* = 23) and unaffected (*n* = 17) by FSF. **(c)** SNPs associated with FSF are interspersed with the signals of selection. Individual SNPs either show a signal of association (black line) or show a high rate of homozygosity (grey line).

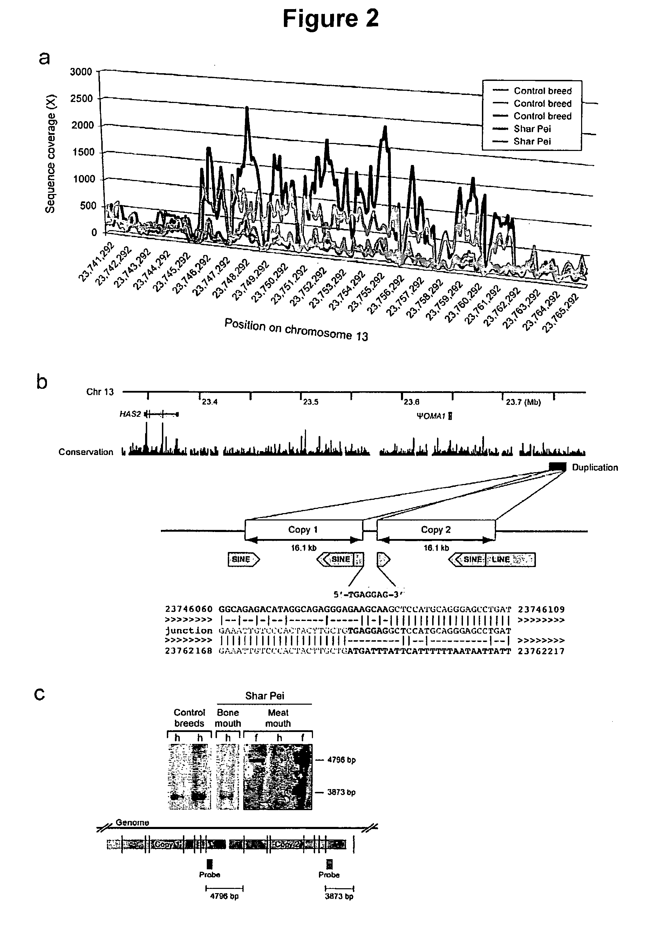

**Figure 2****. A *de novo* duplication with high copy number upstream of *Hyaluronic Acid Synthase 2 (HAS2)* in Shar-Pei. (a)** Targeted resequencing of a 1.5Mb region on chromosome 13 identified a duplication (CanFam2.0 Chr13 23,746,089-23,762,189) with on average 3.5-4.5X higher read coverage in two Shar-Pei (black and grey curves) compared to three control breeds (grey-Standard Poodle, grey-Neapolitan Mastiff and grey- pug). **(b)** The duplication was determined to be 16.1 Kb long with both breakpoints located in repeats (a SINE and a LINE) with an insertion of 7 bp separating different copies. **(c)** Southern blot analysis of control breeds, bonemouths and meatmouth Shar-Pei showed different fragment length for the duplication including both healthy (h, not affected by fever) and fever (f) affected Shar-Pei. Vertical lines in copy 1 and copy 2 indicate the restriction sites of *BsrGI*. The estimated fragment sizes are given to the right.

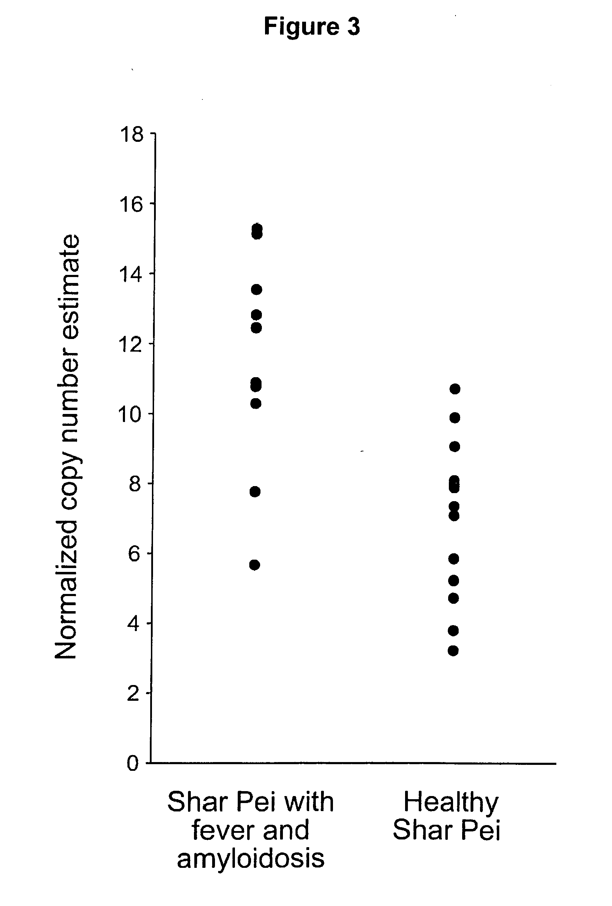

**Figure 3****. Copy number estimates in eleven control breeds and Shar-Pei.** All dogs (control, n=27, Shar-Pei, n=62) were analyzed in triplicate and the range (average +/- SD) plotted. Control dogs have average of two copies (one per chromosome), whereas all Shar-Pei dogs carried the duplication and demonstrated copy number ranging from four to eighteen.

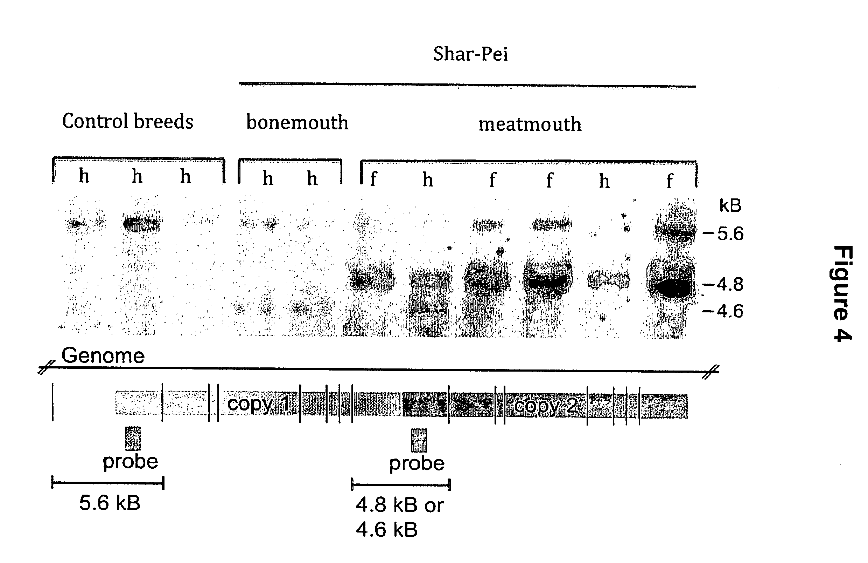

**Figure 4****. A centromeric probe on the BsrGI Southern suggests bonemouth Shar-pei harbor a partial duplication.** This Southern blot shows a smaller band in the bonemouth type in using a centromeric portion, whereas the probe in the telomeric portion of the duplication (Figure 3c) does not. This indicates that bonemouth Shar-Pei carry a partial duplication with a diferent breakpoint. One meatmouth Shar-Pei (known to have several bonemouth Shar-Pei in its pedigree) seemed to be heterozygous for the different copies.

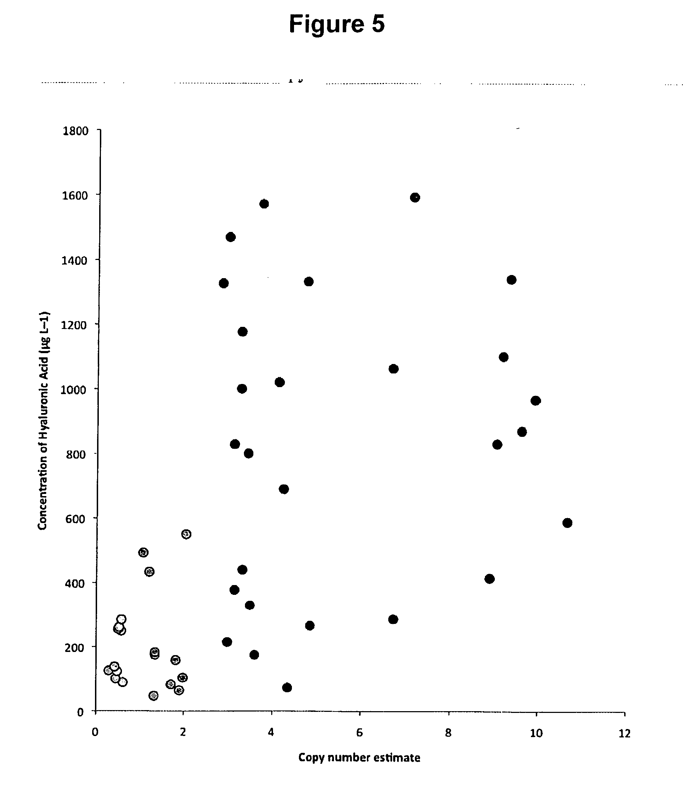

**Figure 5****. Relation between serum hyaluronic acid (HA) concentration and copy number.** None of the control dogs (grey) have the duplication whereas it is present in all Shar-Pei dogs (black, using centromeric probe). All dogs with HA levels higher than 600 µg L⁻¹ carried the duplication although no correlation could be found between HA concentration and copy number.

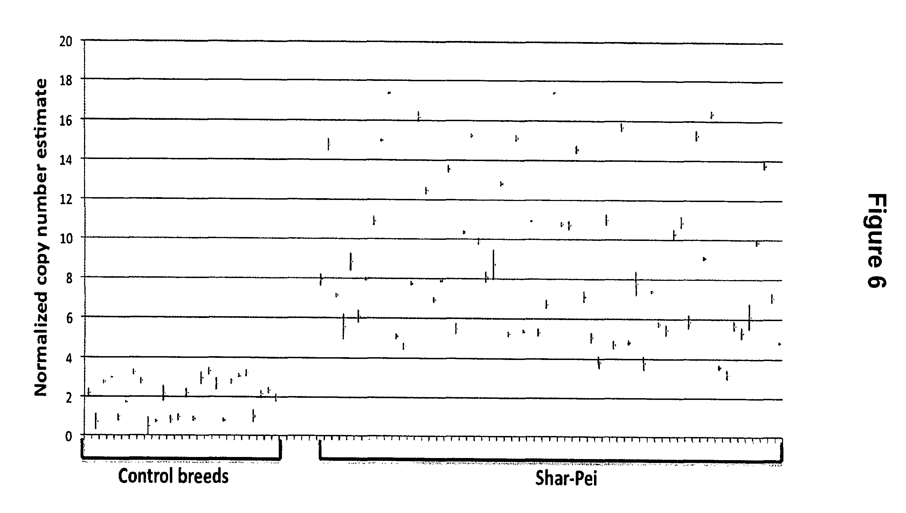

**Figure 6****. A higher copy number of the identified duplication correlates with Familial Shar-Pei Fever**. A significant correlation (P= 0.0006) was seen using an unpaired t-test comparing copy number in unaffected individuals (*n*=15) and individuals severely affected by FSF (*n*=10). Unaffected dogs had no history of unexplained fever and inflammation and had no relatives with a history of recurrent fevers or amyloidosis. Severely affected dogs were all diagnosed with recurrent fever and amyloidosis at post-mortem examination. Based on this limited sample size, most dogs with more than ten copies had fever whereas most dogs with <6 copies did not.

### DETAILED DESCRIPTION OF THE INVENTION

### A. Methodology

Any appropriate method can be used to determine the number of duplications present in the region of nucleic acid located **upstream of the Hyaluronic Acid Synthase 2 (HAS2)** gene on the canine chromosome 13 of a Shar-Pei dog. For example, the number of duplications can be determined by nucleic acid sequencing, denaturing high performance liquid chromatography (DHPLC; Underhill et al, Genome Res., 7:996-1005 (1997)), allele-specific hybridization (Stoneking et al., Am. J. Hum. Genet., 48:370-382 (1991); and Prince et al., Genome Res., 11(1): 152-162 (2001)), allele-specific restriction digests, polymorphism specific polymerase chain reactions, single-stranded conformational polymorphism detection (Schafer et al., Nat. Biotechnol, 15:33-39 (1998)), infrared matrix-assisted laser desorption/ionization mass spectrometry(WO 99/57318), and combinations of such methods.

Genomic DNA can be used to determine the number of duplications present in the region of nucleic acid located **upstream of the Hyaluronic Acid Synthase 2 (HAS2**) gene on the canine chromosome 13 of a Shar-Pei dog. Genomic DNA can be extracted from a biological sample such as peripheral blood samples, hair roots, or tissues (e.g., mucosal scrapings of the lining of the mouth or from renal or hepatic tissue). Any appropriate method can be used to extract genomic DNA from a blood or tissue sample, including, for example, phenol extraction. In some cases, genomic DNA can be extracted with kits such as the QIAamp^{®} Tissue Kit (Qiagen, Chatsworth, CA), the Wizard^{®} Genomic DNA purification kit (Promega, Madison, WI), the Puregene DNA Isolation System (Gentra Systems, Minneapolis, MN), or the A.S.A.P.3 Genomic DNA isolation kit (Boehringer Mannheim, Indianapolis, IN).

Amplification methods such as PCR techniques can be used to determine the number of duplications present in region of nucleic acid located upstream of the Hyaluronic Acid Synthase 2 (HAS2) gene on the canine chromosome 13 of a Shar-Pei dog. For example, a primer pair designed to amplify PCR products containing a duplication breakpoint can be used to determine the number of duplications. Such a primer pair can contain a first primer that anneals upstream of the duplication breakpoint such that extension from that primer proceeds toward the duplication breakpoint and a second primer that anneals downstream of the duplication breakpoint such that extension from that primer also proceeds toward the duplication breakpoint. When a sample contains nucleic acid with the duplication, an appropriately sized PCR product containing the duplication breakpoint can be generated and detected.

The present invention also provides kits that can be used to determine the number of duplications in the region of nucleic acid located upstream of the Hyaluronic Acid Synthase 2 (HAS2) gene on the canine chromosome 13 of a Shar-Pei dog. Such kits can include nucleic acid molecules (e.g., primer pairs or probes), control nucleic acid molecules (e.g., nucleic acid comprising the duplication or apart thereof), DNA aptamers, microarrays, or data analysis software optionally together with any other appropriate reagents, tools, or instructions for performing the methods described herein. Appropriate informational material can be descriptive, instructional, marketing, or other materials that relate to the methods described herein or the use of the reagents for the methods described herein. For example, the informational material can relate to performing a genetic analysis on a Shar-Pei dog and subsequently classifying the horse as being at risk (or not) for developing melanomas. In addition, or in an alternative, the informational material of a kit can be contact information, for example, a physical address, email address, website, or telephone number, where a user of the kit can obtain substantive information about performing a genetic analysis and interpreting the results.

Hybridisation is preferably performed under stringent or highly stringent conditions. "Stringent or highly stringent conditions" of hybridization are well known to or can be established by the person skilled in the art according to conventional protocols. Appropriate stringent conditions for each sequence may be established on the basis of well-known parameters such as temperature, composition of the nucleic acid molecules, salt conditions etc.: see, for example, Sambrook et al. "Molecular Cloning, A Laboratory Manual", CSH Press, Cold Spring Harbor, 1989 or Higgins and Hames (eds.), "Nucleic acid hybridization, a practical approach", IRL Press, Oxford 1985, see in particular the chapter "Hybridization Strategy" by Britten & Davidson. Typical stringent conditions comprise hybridization at 65°C in 0.5xSSC and 0.1% SDS or hybridization at 42°C in 50% formamide, 4xSSC and 0.1% SDS. Hybridization is usually followed by washing to remove unspecific signals. Washing conditions include conditions such as 65°C, 0.2xSSC and 0.1 % SDS or 2xSSC and 0,1% SDS or 0.3xSSC and 0,1 % SDS at 25°C - 65°C.

### B. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Example 1 - Methods

**Samples and diagnostic procedure**. DNA was extracted from blood samples using QIAamp DNA Blood Midi Kit (QIAGEN) or PureLink^{™} Genomic DNA (Invitrogen).

Shar-Pei fever. Shar-Pei dogs were classified *affected* or *unaffected* by FSF based on a strict inclusion and exclusion criteria. Shar-Pei classified as *unaffected* were older than 5 years with no experience of unexplained fever and/or inflammation and also had no close relatives at the grandparental level that could be classified as affected. Shar-Pei classified as *affected* had experienced recurrent episodes of high fever accompanied with inflammation of joints from an early age (< one year old). Affected individuals who were subjected to post-mortem examination were sub-classified as *severely affectecl* if depositions of amyloid in kidneys and/or liver were detected (amyloidosis).

Hyaluronanosis. Serum hyaluronic acid (HA) concentration was used as a proxy for hyaluronanosis but no cut-off value was established. HA measurements were performed using the Hyaluronan ELISA kit (Echelon Biosciences INC) according to the manufacturer's instructions. The absorbence was read at 405 nm, and a semi-log standard curve was used to calculate hyaluronic acid concentrations.

**Homozygosity and genome-wide association mapping**. A whole genome scan was performed with two runs, 27K (v1) and 50K (v2) canine Affymetrix SNP chips. Results were called using Affymetrix's snp5-geno-qc software. The 50K array was used when the rate of heterozygosity was calculated for US Shar-Pei separately and for a reference group of 24 other breeds. The ratio of heterozygosity in 10 SNP (≈1 Mb) sliding windows between the two groups was used as a measure of relative heterozygosity. To look for regions of homozygosity within the Shar Pei genome only, the software package PLINK16 was used to search for within individual runs of homozygosity. This was performed both for the 50 K array with 50 US Shar-Peis and replicated for 37 Spanish Shar-Peis using 22,362 SNPs genotyped with the Illuninas CanineSNP20 BeadChip. These data were collected with an Illumina BeadStation scanner and genotypes were scored using GenomeStudio. Regions of homozygosity were defined if shared across all Shar-Pei samples. A case-control association analysis using 17.227 SNPs common to both the 27K and 50K arrays (n= 17 227, MAF > 0.05, call rate > 75%) was performed in Shar-Pei classified as affected (n=23) or unaffected (n=17) by Shar-Pei fever. The software package PLINK16 was used for the analyses and to ensure genome-wide significance p-values were corrected for multiple testing. Values used are the max (T) empirical p-values obtained after 100 000 permutations.

**Targeted resequencing.** Target capture of the 1.5Mb homozygous candidate region (CanFam 2.0 Chr13: 22,937,592-24,414,650) was performed by using a 385K custom-designed sequence capture array from Roche NimbleGen. Hybridization library preparation was performed as following: Genomic DNA (15-20µg) was fragmented using sonication; blunting of DNA fragments using T4 DNA Polymerase, Klenow Fragment and T4 Polynucleotide Kinase; adding A-overhangs using Klenow Fragment exo⁻ and ligation of adaptors using T4 DNA Ligase with Single-read Genomic Adapter Oligo Mix (Illumina). All enzymes were purchased from Fermentas and used following manufacturers instructions. Purification steps were performed using QIAquick PCR Purification Kit (QIAGEN). Hybridization was performed following the manufacturers instructions without amplification of the fragment library prior to hybridization. Eluted captured DNA and uncaptured libraries were amplified using Phusion High Fidelity PCR Master Mix (Finnzymes) and the SYBR Green PCR Master Mix (Applied Biosystems) was used to estimate the relative fold-enrichment. Capture libraries with the estimated enrichment-factor of >200 were sequenced using Genome Analyzer (Illumina) and obtained sequences were aligned to reference dog sequence¹⁹ of the targeted region using Maq assembly²⁰.

**Polymerase Chain Reaction (PCR) and Sanger sequencing**. All primers used were designed using Primer3²¹ and are listed in **Table 2.** PCRs and Sanger Sequencing were performed to investigate putative mutations (five SNPs and one indel) and were carried out with 20 ng genomic DNA using AmpliTaq Gold ® DNA Polymerase (Applied Biosystems) following the manufacturers instructions. The amplification of the copy number variant (CNV) breakpoints was performed with 400 ng of DNA and a Long-range PCR with Expand Long Template PCR System Mix 1 (Roche), cloned using Zero Blunt TOPO Cloning Kit (Invitrogen) and plasmid DNA prepared using QIAprep Spin Miniprep Kit (QIAGEN). PCR products and plasmids were sequenced using capillary electrophoresis 3730x1 (Applied Biosystems), aligned and analyzed using CodonCode Aligner version 2.0.6 (CodonCode).

**Southern blot analysis**. Four micrograms of genomic DNA from each sample was digested with BsrGI (New England BioLabs) and separated on a 0.7% agarose gel. Hybridizations and blot visualization were performed with the Gene Images AlkPhos Direct Labelling and Detection System (GE Healthcare) following the manufactures instructions using a 910bp probe designed to chromosome 13 (targeting CanFam 2.0 Chr13: 23,746,12-23,747,522). Hyperfilm ECL (Amersham) films were developed following exposure to the labeled blot for one hour.

**Real time PCR.** Relative fold-enrichment was performed using the comparative ΔC_{T}-method where an assay designed within the CNV (SP Duplication Inside) was normalized to an assay flanking the CNV (SP Duplication Outside). Fast Real-Time PCR was performed using TaqMan ® Universal PCR Master Mix (Applied Biosystems) and a 7900 HT Fast Real-Time PCR system (Applied Biosystems).

### Example 2 - Identification of candidate loci for the breed-specific phenotype hyaluronanosis.

The present inventors screened the genome for signatures of selective sweeps. Selective sweeps can be recognized as long chromosomal segments with a low degree of heterozygosity within populations¹⁴. Using 50,000 single nucleotide polymorphisms (SNPs) distributed throughout the dog genome the level of heterozygosity in 10 SNP windows was compared between a set of Shar-Pei (n = 50, all from the US) and the average of 24 other canine breeds (n = 230). On four chromosomes (Cfa 5, 6, 13 and X) the reduction in heterozygosity in Shar-Pei was greater than 4-fold the average of control breeds (**Figure 1a**). The strongest signal of reduced heterozygosity appeared within a 3.7 Mb stretch on chromosome 13 (CanFam 2.0 Chr13: 23,487,992-27,227,623) near the HAS2 gene, where almost complete homozygosity was observed in Shar Pei (**Figure 1c**). Here the reduction in heterozygosity was greater than 10-fold in Shar-Pei and several smaller regions showed complete homozygosity. The same region was overwhelmingly homozygous in 37 Spanish Shar-Pei samples and was partially the same as reported in a recent study¹⁵. The strong signal, together with the known function of HAS2, made this region an obvious candidate region for harboring the hyaluronanosis mutation.

### Example 3 - Mapping of the susceptibility loci for Familial Shar-Pei Fever (FSF)

The present inventors performed a genome-wide association to map the susceptibility loci for FSF, using Shar-Pei strictly classified into FSF affected (*n*=23) and unaffected (*n*=17). Interestingly, five SNPs were significantly associated (best SNP p_{raw}=1.5x10⁻⁶, p_{genome}=0.01 based on 100,000 permutations; software package Plink¹⁶), all on chromosome 13 (CanFam 2.0 Chr13: 27,9- 30,7 Mb, **Figure 1b**). When comparing the signals of association and the regions of low heterozygosity, they appear interspersed so that individual SNPs are either part of homozygous regions or signals associated with FSF (**Figure 1c**). Hence, it is difficult to determine exactly where the strongest association resides.

### Example 4 - Identification of a duplication upstream of the Hyaluronic Acid Synthase 2 (HAS2) gene in Shar-Pei

Using targeted sequence capture technology, the present inventors resequenced 1.5 Mb around and upstream of *HAS2* (CanFam 2.0 Chr13: 22,937,592- 24,414,650) in two Shar-Pei and three control dogs from other breeds. Dogs were chosen based on HA serum levels, a proxy measurement for hyaluronanosis. The HA levels were three to four-fold higher in Shar-Pei than in control dogs (**Table 1a**).

The obtained sequences were mapped to the boxer reference sequence providing at least 5X coverage for 96-98% of the resequenced region in each individual. After masking repetitive sequences we found -670 indels and ∼1,500 SNPs in each dog (**Table 3**) as well as a duplication that was present in high copy number in the two Shar-Pei (**Figure 2a**).

**Table 1a. Dogs used in resequencing and their Hyaluronic acid concentration**

| **Breed** | | **Total included** | **hyaluronic acid¹** |
|---|---|---|---|
| **Shar-Pei** | | 2 | |
| | Meatmouth type | 2 | 1484, 2052 |
| **Control breeds** | | 3 | |
| | Standard Poodle | 1 | 151 |
| | Pug | 1 | 492 |
| | Neapolitan Mastiff | 1 | 351 |

| | | | |
|---|---|---|---|
| ¹ Serum concentration of hyaluronic acid expressed in µg L⁻¹ | | | |

**Table 1b. Dogs used in the Southern blot analysis**

| **Breed** | | **Number of individuals** | **Affection status Familial Shar-Pei Fever** |
|---|---|---|---|
| **Shar-Pei** | | **8** | |
| | bonemouth type | 2 | Unaffected |
| | meatmouth type | 2 | Unaffected |
| | meatmouth type | 4 | Affected |

| **Control breeds** | | **3** | |
|---|---|---|---|
| | German Shepherd | 1 | Unaffected |
| | Eurasian | 1 | Unaffected |
| | Rough coated collie | 1 | Unaffected |

**Table 1c. Dogs used in quantitative PCR experiments in the search of the presence of duplication and association to hyaluronosis and Familial Shar-Pei Fever (FSF)**

| **Breed** | **Total included** | **hyaluronic acid¹** |
|---|---|---|
| **Control breed** | **71** | **158** |
| **Shar-Pei (meatmouth type)** | **56** | **831** |
| FSF² | 12 | |
| FSF+A | 10 | |
| H | 11 | |
| h | 11 | |
| Uncertain diagnosis | 12 | |
| **Shar Pei (bonemouth type)** | **4** | **175** |
| FSF | 0 | |
| FSF+A | 0 | |
| H | 4 | |
| h | 0 | |
| Uncertain diagnosis | 0 | |

| | | |
|---|---|---|
| ¹ Median level of hyaluronic acid expressed in µg L⁻¹ ² Familial Shar-Pei Fever (FSF) status FSF, Shar-Pei reported to have experienced unexplained fever, FSF+A, Shar-Pei reported to have experienced frequent episodes of unexplained fever and positive when testing for amyloidosis, H, Shar-Pei, older than five years, reported to never have experienced unexplained feverepisodes and have no first degree relatives diagnosed with FSF or amyloidosis, h, Shar-Pei reported to never have experienced unexplained fever episodes. | | |

**Table 2. Primers and probes**

| | **Forward primer 5'-3'** | **Reverse primer 5'-3'** | **T_{A}¹** |
|---|---|---|---|
| **Mutation screening** | | | |
| Conserved SNP1 | | | 60 |
| Conserved SNP2 | | | 60 |
| Conserved SNP3 | | | 60 |
| Conserved SNP4 | | | 60 |
| Conserved SNP5 | | | 60 |
| Conserved Indel | | | 59 |

| **Identification of duplication breakpoint** | | | |
|---|---|---|---|
| Duplication breakpoint | | | 65 |

| **Quantitative PCR** | | | |
|---|---|---|---|
| SP Duplication Inside | | | |
| SP Duplication Outside | | | |

| **Southern Blot analysis** | | | |
|---|---|---|---|
| Cetromeric probe | | | 60 |
| Teleomeric Probe | | | 60 |

| | | | |
|---|---|---|---|
| ¹ Annealing temperature in °C | | | |

**Table 3. Summary statistics of the targeted resequencing compared to full or repeat-masked genome sequence (CanFam 2.0 Chr13: 22,937,592-24,414,650)**

| | | **Full Reference Sequence in Target Region** | | | **Repeat-masked Reference Sequence in Target Region** | | |
|---|---|---|---|---|---|---|---|
| **breed** | **No of reads** | **% mapped¹** | **Coverage²** | **No of SNPs³** | **% mapped¹** | **Coverage²** | **No of SNPs³** |
| Nea. Mastiff | 11,011,789 | 92 | 246 | 2773 | 60 | 256 | 1380 |
| Pug | 9,868,613 | 74 | 178 | 3381 | 45 | 171 | 1630 |
| Poodle | 8,672,727 | 79 | 165 | 2646 | 48 | 161 | 1356 |
| Shar-Pei A | 10,789,354 | 90 | 236 | 3399 | 58 | 245 | 1791 |
| Shar-Pei B | 9,715,905 | 75 | 176 | 3343 | 47 | 176 | 1666 |

| | | | | | | | |
|---|---|---|---|---|---|---|---|
| ¹ Proportion of reads aligned the dog reference sequence (CanFam 2.0) ² Average number of sequences covering each base position ³ Number of SNPs identified in each resequenced individual compared to the reference genome (CanFam 2.0) | | | | | | | |

Six mutations (four SNPs and one indel located in conserved elements, and the duplication) were tested for correlation to the hyaluronanosis phenotype using 13 additional dogs from seven breeds. Only the duplication was concordant with phenotype, thus allowing us to exclude the other five variants. The duplication was determined to be 16.1 Kb long (CanFam 2.0 Chr13: 23,746,089-23,762,189) (SEQ ID NO: 1) with breakpoints located in repeats (a SINE at the centromeric end and a LINE at the telomeric end) and individual copies were separated by a seven base pair sequence (SEQ ID NO: 24) (**Figure 2b**).

### Example 5. Confirmation of the duplication in Shar-Pei

The present inventors confirmed the presence of a *de novo* duplication in Shar-Pei by performing Southern Blot and/or quantitative PCR in 74 dogs from 27 other breeds, none of which had the duplication (**Table 1b and 1c,** **Figure 3**) Interestingly, with a telomeric probe on the Southern Blot (**Figure 2c**), bonemouth Shar-Pei did not appear to have a duplication, but with a centromeric probe an extra band was seen (**Figure 4**), suggesting that the bonemouth type might carry a partial duplication with a different breakpoint. In addition, an intensity difference was seen between different Shar-Pei (**Figure 4**), suggesting variation in copy number for the duplication.

### Example 6. Association between high copy number of the duplication and severe Familial Shar-Pei Fever

To more carefully quantify how copy number might influence the severity of hyaluronanosis and/or FSF, the present inventors performed quantitative PCR in 62 Shar-Pei, who were affected by hyaluronanosis to different degrees based on serum HA measurements and also classified as affected or unaffected by FSF. The number of copies was estimated as the relative fold enrichment (ΔCt) between amplicons within and outside the duplication. No clear association was detected between serum level of HA in Shar-Pei and duplication copy number (**Figure 5**), although the mean HA level in Shar-Pei with ≥ 6 copies was 905 ± 403 ug/L, while Shar-Pei with fewer copies had an mean concentration of 770 ± 494 ug/L and control breeds had HA serum levels of 206 ± 145 ug/L. Interestingly, the three bonemouth Shar-Pei had between 73 and 266 ug/L, within the normal range. The difficulties to correlate serum levels of HA with other clinical and biomedical parameters have been seen in many human studies, where no or only weak correlations have been obtained.^{17,18} Notably, we obtained a significant association (P = 0.0006) between higher copy number and severe FSF, in dogs that had experienced frequent episodes of fever and inflammation and had histologically confirmed amyloidosis (*n*=10) in contrast to Shar-Pei that were themselves unaffected by FSF and that lacked first degree relatives with fever (*n*=15) (**Figure 6**). The observed association, despite the very high homozygosity in this region, strongly suggests that a high copy number of this duplication is not just a genetic marker for FSF but is causally related to the development of disease

### REFERENCES

1. Kastner, D.L., Aksentijevich, I., Goldbach-Mansky, R. Autoinflammatory Disease reloaded: A clinical perspective. Cell 140, 784-790 (2010).
2. Rivas, A.L., Tintle, L. Kimball, E.S., Scarlett, J., Quimby, F.W. A canine febril disorder associated with elevated interleukin-6. Clinical Immunology and Immunopathology 64, 36-45 (1992).
3. Zanna et al. Hereditary cutaneous mucinosis in Shar-Pei dogs is associated with increases hyaluron synthase-2 mRNA transcription by cultured dermal fibroblasts. Veterinary Dermatology 20, 377-382 (2009).
4. Laurent, T.C. & Fraser, J.R.E. Hyaluronan. FASEB J. 6, 2397-2404 (1992).
5. Yamasaki, K. et al. NLRP3/Cryopyrin is necessary for Interleukin-1β (IL-β) release in response to hyaluronan, an endogenous trigger in inflammation in response to injury. Journal of Biological Chemistry 284 12762-12771 (2009)
6. Hascall, VC. et al. Intracellular hyaluronan: a new frontier for inflammation? Biochimica et Biophysica Acta 1673, 3-12 (2004).
7. Fraser, J.R., Laurent, T.C., Laurent, U.B. Hyaluronan: its nature, distribution, functions and turnover. Journal of Internal Medicine 242, 27-33 (1997).
8. Zanna, G. et al. Cutaneous mucinosis in Shar-Pei dogs is due to hyaluronic acid deposition and is associated with high levels of hyaluronic acid in serum. Veterinary Dermatology 19, 314-318 (2008).
9. Ramsden, C.A. et al. A new disorder of hyaluronan metabolism associated with generalized folding and thickening of the skin. The Journal of Pediatrics 36, 62-68 (2000).
10. Lindqvist, U. & Laurent, T.C. Serum hyaluronan and aminoterminal propeptide of type III procollagen: variation with age. Scandinavian Journal of Clinical Laboratory Investigations 52, 613-621 (1992).
11. Itano, N. & Kimata, K. Mammalian hyaluronan synthases. IUBMB Life 54, 195-199 (2002).
12. Weigel, P.H., Hascall, V.C., Tammi, M. Hyaluronan synthases. Journal of Biological Chemistry 272, 13997-4000 (1997).
13. Stojanov, S. & Kastner D.L. Familial autoinflammatory diseases: genetics, pathogenesis and treatment. Current Opinion in Rheumatology 17:586-599 (2005).
14. Smith, J.M. & Haigh, J. The hitchhiking effect of a favorable gene. Genetic Research 23:23-35 (1974).
15. Akey, J.M. et al. Tracking footprints of artificial selection in the dog genome. PNAS 19, 1160-1165 (2010).
16. Purcell, S. et al. PLINK: a tool set for whole-genome association and population-based linkage analyses. American Journal of Human Genetics 81, 559-575 (2007).
17. Goldberg, R.L., Huff, J.P, Lenz, M.E., Glickman, P., Katz, R. Thonar, E.J-M.A. Elevated plasma levels of hyaluronate in patients with osteoarthritis and rheumatoid arthritis. Arthritis Rheum 34, 799-807 (1991).
18. Hedin, P.J, Weitoft, T., Hedin, H., Engström-Laurent, A., Saxne, T. Serum concentration of hyaluronan and proteoglycans in joint disease. Lack of association. J. Rheumatol 18, 1601-1605 (1991).
19. Lindblad-Toh, K. et al. Genome sequence, comparative analysis and haplotype structure of the domestic dog. Nature 438, 803-819 (2005).
20. Heng, L., Ruan, J., Durbin, R. Mapping short DNA sequencing reads and calling variants using mapping quality scores. Genome research 18, 1851-1858 (2008).
21. Rozen, S & Skaletsky, H. Primer3 on the WWW for general users and for biologist programmers. Bioinformatics Methods and Protocols: Methods in Molecular Biology. pp 365-386 (Humana Press, Totowa, New Jersey, USA, 2000).

## Claims

1. A method for testing a Shar-Pei dog for its genetic predisposition to develop Familial Shar-Pei fever which method comprises, or consists essentially of, obtaining sequence information from the region of nucleic acid located upstream of the Hyaluronic Acid Synthase 2 (HAS2) gene comprising the nucleic acid sequence consisting of bp 22,937,592 to bp 24,414,650 on the canine chromosome 13 to determine the copy number of a 16.1 kb duplication consisting of the nucleic sequence SEQ ID NO:1 or a sequence having more than 95 % identity to SEQ ID NO:1 present in this region, wherein the copy number of said duplication is indicative of the risk that the dog will develop Familial Shar-Pei fever.

2. The method according to claim 1, wherein a copy number of >10 of the duplication indicates a high risk for Shar-Pei fever, a copy number of <5 indicates a low risk, and a copy number between 5-10 indicates an intermediary risk.

3. The method according to claim 1, which comprises the use of a nucleic acid probe comprising a sequence present in a duplication on the canine chromosome 13 corresponding to the nucleic acid sequence SEQ ID N0:1, and wherein the nucleic acid probe is capable of hybridizing to a part of the nucleic acid sequence SEQ ID NO:1, or a sequence complementary thereto, under stringent conditions.

4. An isolated nucleic acid primer pair comprising a first primer and a second primer, wherein each of the first and second primers comprises a sequence present in a duplication on the canine chromosome 13 corresponding to the nucleic acid sequence SEQ ID NO:1, and wherein the primer pair is capable of amplifying a nucleic acid containing at least a part of said duplication.

5. The nucleic acid primer pair according to claim 4, wherein at least one primer is selected from SEQ NO:2, SEQ NO:3, SEQ NO:4, SEQ ID NO:5, SEQ NO:6, SEQ NO:7, SEQ NO:8, SEQ NO:9, SEQ ID NO:10, SEQ NO:11, SEQ NO:12, SEQ NO:13, SEQ NO:14, SEQ NO:15, SEQ NO:16, SEQ NO:17, SEQ NO:18, SEQ NO:19, SEQ NO:20 SEQ NO:21, SEQ NO:22 and SEQ ID NO:23.

6. An isolated nucleic acid primer pair comprising a first primer and a second primer, wherein each of the first and second primers comprises a sequence present in a duplication on the canine chromosome 13 corresponding to the nucleic acid sequence SEQ NO:1, and wherein the primer pair is capable of amplifying a nucleic acid containing a breakpoint of the duplication

7. The nucleic acid primer pair according to claim 6, wherein at least one primer is selected from SEQ NO:14, and SEQ NO:15

8. The method according to claim 1, which comprises the use of a nucleic acid primer pair according any of claims 4 to 7.

## Patentansprüche

1. Verfahren zum Testen eines Shar-Pei-Hundes auf seine genetische Prädisposition hin Familiäres Shar-Pei-Fieber zu entwickeln, wobei das Verfahren umfasst oder im Wesentlich besteht aus Erhalten von Sequenzinformation von der Nukleinsäureregion, welche stromaufwärts von dem Hyaluronsäuresynthase 2 (HAS2)-Gen lokalisiert ist, umfassend die Nukleinsäuresequenz bestehend aus bp 22.937.592 bis bp 24.414.650 auf dem Hundechromosom 13, um die Kopienzahl von einer 16,1 kb Duplikation bestehend aus der Nukleinsäuresequenz SEQ ID NR:1 oder einer Sequenz, welche in diesem Bereich vorhanden ist, die mehr als 95 % Identität zu SEQ ID NR:1 aufweist, zu bestimmen, wobei die Kopienzahl von der Duplikation indikativ für das Risiko ist, dass der Hund Familiäres Shar-Pei-Fieber entwickeln wird.

2. Verfahren nach Anspruch 1, wobei eine Kopienzahl von > 10 der Duplikation ein hohes Risiko für Shar-Pei-Fieber anzeigt, eine Kopienzahl von < 5 ein geringes Risiko anzeigt und eine Kopienzahl zwischen 5-10 ein mittleres Risiko anzeigt.

3. Verfahren nach Anspruch 1, welches die Verwendung einer Nukleinsäuresonde umfasst, umfassend eine Sequenz, welche in einer Duplikation auf dem Hundechromosom 13 vorliegt, entsprechend der Nukleinsäuresequenz SEQ ID NR:1 und wobei die Nukleinsäuresonde fähig ist unter stringenten Bedingungen mit einem Teil von der Nukleinsäuresequenz SEQ ID NR:1 oder einer Sequenz komplementär hierzu zu hybridisieren.

4. Isoliertes Nukleinsäureprimerpaar, umfassend einen ersten Primer und einen zweiten Primer, wobei jeder von den ersten und zweiten Primern eine Sequenz, welche in einer Duplikation auf dem Hundechromosom 13 vorliegt, entsprechend der Nukleinsäuresequenz SEQ ID NR:1 umfasst und wobei das Primerpaar fähig ist, eine Nukleinsäure, welche mindestens einen Teil von der Duplikation enthält, zu amplifizieren.

5. Nukleinsäureprimerpaar nach Anspruch 4, wobei mindestens ein Primer ausgewählt ist aus SEQ ID NR:2, SEQ ID NR:3, SEQ ID NR:4, SEQ ID NR:5, SEQ ID NR:6, SEQ ID NR:7, SEQ ID NR:8, SEQ ID NR:9, SEQ ID NR:10, SEQ ID NR:11, SEQ ID NR:12, SEQ ID NR:13, SEQ ID NR:14, SEQ ID NR:15, SEQ ID NR:16, SEQ ID NR:17, SEQ ID NR:18, SEQ ID NR:19, SEQ ID NR:20, SEQ ID NR:21, SEQ ID NR:22 und SEQ ID NR:23.

6. Isoliertes Nukleinsäureprimerpaar umfassend einen ersten Primer und einen zweiten Primer, wobei jeder von den ersten und zweiten Primern eine Sequenz, welche in einer Duplikation auf dem Hundechromosom 13 vorliegt, entsprechend der Nukleinsäuresequenz SEQ ID NR:1 umfasst und wobei das Primerpaar fähig ist, eine Nukleinsäure, welche eine Bruchstelle (breakpoint) von der Duplikation enthält, zu amplifizieren.

7. Nukleinsäureprimerpaar nach Anspruch 6, wobei mindestens ein Primer ausgewählt ist aus SEQ ID NR:14 und SEQ ID NR:15.

8. Verfahren nach Anspruch 1, welches die Verwendung von einem Nukleinsäureprimerpaar nach jedem der Ansprüche 4 bis 7 umfasst.

## Revendications

1. Un procédé de test d'un chien Shar-Pei en ce qui concerne sa prédisposition génétique à développer la fièvre familiale des Shar-Pei, lequel procédé comprend, ou consiste essentiellement en, l'obtention d'informations de séquence de la région de l'acide nucléique située en amont du gène de l'Acide Hyaluronique Synthase 2 (HAS2) comprenant la séquence d'acide nucléique allant de 22.937.592 bp à 24.414.650 bp sur le chromosome canin 13 pour déterminer le nombre de copies d'une duplication 16,1 Kb consistant en la séquence nucléique SEQ ID NO:1 ou en une séquence présentant une identité de plus de 95% avec la SEQ ID NO:1 présente dans cette région, le nombre de copies de ladite duplication indiquant le risque que le chien développe la fièvre familiale des Shar-Pei.

2. Le procédé selon la revendication 1, dans lequel un nombre de copies de >10 de la duplication indique un risque élevé de fièvre des Shar-Pei, un nombre de copies de <5 indique un faible risque, et un nombre de copies situé entre 5 et 10 indique un risque intermédiaire.

3. Le procédé selon la revendication 1, qui comprend l'utilisation d'une sonde d'acide nucléique comprenant une séquence présente dans une duplication sur le chromosome canin 13 correspondant à la séquence d'acide nucléique SEQ ID NO:1, et dans lequel la sonde d'acide nucléique est capable de s'hybrider à une partie de la séquence d'acide nucléique SEQ ID NO:1, ou à une séquence complémentaire de celle-ci, sous des conditions strictes.

4. Une paire d'amorces isolée d'acide nucléique comprenant une première amorce et une seconde amorce, dans laquelle chacune des première et seconde amorces comprend une séquence présente dans une duplication sur le chromosome canin 13 correspondant à la séquence d'acide nucléique SEQ ID NO:1, et dans lequel la paire d'amorces est capable d'amplifier un acide nucléique contenant au moins une partie de ladite duplication.

5. La paire d'amorces d'acide nucléique selon la revendication 4, dans laquelle au moins une amorce est choisie parmi SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO:10., SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:22, et SEQ ID NO:23.

6. Une paire d'amorces isolée d'acide nucléique comprenant une première amorce et une seconde amorce, dans laquelle chacune des première et seconde amorces comprend une séquence présente dans une duplication sur le chromosome canin 13 correspondant à la séquence d'acide nucléique SEQ ID NO:1, et dans laquelle la paire d'amorces est capable d'amplifier un acide nucléique contenant un point de rupture de la duplication

7. La paire d'amorces d'acide nucléique selon la revendication 6, dans laquelle au moins une amorce est choisie parmi SEQ ID NO:14, et SEQ ID NO:15.

8. Le procédé selon la revendication 1, qui comprend l'utilisation d'une paire d'amorces d'acide nucléique selon l'une quelconque des revendications 4 à 7.


### SEQUENCE LISTING

<110> Lindblad-Toh, Kerstin Olsson, Mia Tintle, Linda
<120> GENETIC RISK ASSESSMENT FOR SHAR-PEI FEVER
<130> x
<150> US 61/356,230
   <151> 2010-06-18
<160> 24
<170> PatentIn version 3.5
<210> 1
   <211> 16101
   <212> DNA
   <213> Canis familiaris
<400> 1
   gctccatgca gggagcctga tgtggcactt gatcctggga ctccaggatc atgccctgag      60
   ctgaaggcag gtgcttaccc gctgagccac ccaggcatcc caataaaatc ttttaaaaat     120
   aaataaataa aatactcttg gtgtaatgtt atgaaactgg actatttata cataatcgta     180
   tagtataaga tttaggcagt gatttctaaa ttgcccatat agtaaaaatt atatataaac     240
   tatattatat ataatatatg acatataaag aaatgttaca tatagatata gataactaga     300
   tatatgtctt cctgaatctt aatacaatga gtatttctaa atgtttacat ttgaaaaaaa     360
   tccacaggaa attatgagga acacttaaga ttgagaaaca ttgcccaaaa ggatattaat     420
   ctgcattgat atttctattg tggaataaca aaaattgaag gatgggacac attagtgtat     480
   gttaaatcac ttcgaaagat agatgcttat taaatatgtg taatagatca tgccttgcta     540
   cctgaaagtt ggaataatgc aaacttattc ctttaaataa agactattca ttacaagtaa     600
   tttttttaaa acttgacaat ggtttgatgt tagaatgctt ttaatttcaa gtaaatccat     660
   ccaacagtgg ctgaaacaaa taagggattg cttttttgtg cctgtctctt caaattacaa     720
   ggcatctgga gagagctagc tactggcagt ggttcaacag ctcagctgca tcacggtgac     780
   tgtcagatct tctcagcttt tacttacata tatacaaggt gaattgcaaa cttaggcatc     840
   atacccatgg attttaaggc agaaggaaag agcagtagct tcggggtact ctctaaagca     900
   aacgctcttc aaaaagcctc tgcttatgtt tcatttgtct agtgctgtgt taaaaagcta     960
   tccttggata aaagggaggc tggggagatc ctaaaaatcc ctaggcattg taaatgaaat    1020
   acctaaagga aataaagtgc tctacagact gcctaataga taataagact gccaaaagag    1080
   gcgttataaa accaaaaata acaacaatag caacaaaacc ccaaaaaaca aaaaactgta    1140
   ctcaggattt tcagttgtat ggtagcaata tctttcattg tatcaccatg aaaattaaat      1200
   aaaacaattt cagaaaaatt tattgagctc ttacagatat caggtagcct tggctttgga      1260
   gacaagttag aatagtacag agtctctatt cacagagctc actttctaat agggaaactg      1320
   agtgtaaatc aatagattca agaattgata aatccaggaa gaagacagaa gtaggttaaa      1380
   ttgaagaaag ggatttatgg ttgccaggga gagtggtggg taacaatggt tagggaagat      1440
   ctttctgagg cagaggaaga ccagattatt aggaagtagg acattcaaac aaaagtttta      1500
   tccaggaaag ggggttggat tttgtgtgta aaaacaaatt taagtgagtg tggctaaaaa      1560
   tcaattcata gagtaaagag tagtcatctg agccaactca ggcaaaccct gcctacaatt      1620
   aggggcctat atttgggtat agtggaacac cattggttaa tattttaatc agtggggttt      1680
   atgatttaag gaatgctttt tttttagaga taatgtttat tttattttac tttttaagat      1740
   ttttatttat ttatgagaga cacagaggga gagagagaga gaggccgaga cacaggcaga      1800
   gggagaagca ggctccattc agggagcccc atgtgggact caatcccggg tctccaggat      1860
   cacgccttgg gtcaaaggca ggcgccaaac tgctgagcca cccagggatc tctaaggaat      1920
   gctttttaaa aagataacca tagcttccct gtagaagata gaataaaaga atgcaatatt      1980
   agaggcaggg agaccaatat agctgggaaa tagaaatggc tcagattgta gaagtggtaa      2040
   taattagatt caagatatat tttgaaggta tatcctcctg gatcttctat ttattagatg      2100
   tacactatga aggaagtaga tgaattcaag ttgacttcta gattttcaga caaaacaact      2160
   gagtgaaaaa gagtgtcatt tactgagatt agtgaaagct tgtaggaaga gtcatttggg      2220
   ggcataggga ggaaagtagc tctgctcttg ccaatataat tttgatgtgc gtatagtact      2280
   cccaaaagca agcacaaaat agagggtatg catttatgac tatgcagctc aggagagatt      2340
   ttttagatta aaccattcaa accactgtag cagggcacaa aagtgaaaaa gaactgtgtg      2400
   cttgaaatgg tcctaaccat caaccattaa taaaagaaaa ggagtccaca agtctagaaa      2460
   tgtgggcaaa gattcacaac tgcagacagg ctcttcctga aatctgtagc atagaactaa      2520
   cgatgaggac acaaaggtgg tcaaccacag tgaaacccac aaaagtagaa taaaaaatgt      2580
   gtgaagtgtg tatgtaaaca cacgaaaagc aaagttgaaa gcatttttcc agtgactgtg      2640
   atagaagagt ctccagtgaa ttgctcatgt aataaagtca atggcattag aagaggaatt      2700
   gtctccgtgt tggtccaggc ctttcgtcag tttgaatagt tagtgcacaa catttcatgt      2760
   gcagttgtca tgtcgcaaag ggacaccacg tgacttgtgg gacatctcta tcaagaaacc      2820
   aacgaatcac tccacagatg ctgttcagag tgcttgcttg ctctgatggc tgccctacag      2880
   tgtctactta aaggcacctc agtggatccg ccacacaaac tgactagcta ctaaaagaat      2940
   acttgtgaat gatgtgaaaa aaaatcccaa aacttctgtt caaaccaatt tggttgatcc 3000
   tttcatattc tgaagcacac aaagaaggtt tcctgggagt aatttctgtt cagtgtccac 3060
   atttcataac atttattgga tttatgcaat tctcttgact cccccctgcc cccaaagcac 3120
   tttcatatgt gggcttactt tagactcagg acacttcacg gagatggata ggatagccat 3180
   attcatctgc ttttctgagc cagaagcttc ttcagaatgg gaaatactta aactatagcc 3240
   aaagaaacat ccattaatga cctgtggagg accggtttca gttggaacta cctgcatctg 3300
   gtcatatcct aacaccctaa aagtatgttc tttggcaaaa ttacttccaa tattagggat 3360
   ttggaattgc agccaaacat tcttacatat acagatatac tgacctggca gagatgttac 3420
   aggaaggggg tgaaatgtaa atccatacgg cagctataca atttacgtat tttgcaagat 3480
   gatgtattcc tccttaggaa attgagaaat aatagttact tcaaattatc ctcgaagatc 3540
   atactttatt aaaactaact gaacgattaa atttataata tttttctcct atacaataat 3600
   taaaagctgt ggctgcttct caaaaaaaaa aggagggggg aagaggggga agagaagaag 3660
   aaaataaatt tcaaaaagat tttgcctttg tttagatatt tttgtggtac ctttcattaa 3720
   tggccataaa taaaaacaat tcctgattat ttttcttcaa agttttaatt tttctcttta 3780
   tttaattata attagttgtg atcttcattt tctttcatat gaataagaga aagaaatttt 3840
   gaaagaaatt tttgaaattt ggaaagaaag aaaacagcac tacagagaaa ctgcatactg 3900
   catgtcttct tcttggaaat caatatttaa acaaatcaac ccatagacat atagcttgaa 3960
   aattacagat ttgtttttaa gcttcctgtt tttaagcctc taagactgag gtaccagaag 4020
   cacagttagc tattgaaaaa tctttacctc agaaaaaaaa atttattccc tgtcctcttt 4080
   tgccacacag tttgcgtgtg tttatgtgtg ttttttaatg tagagcccag ttgtgacata 4140
   tttgttttct tttgtattct cccaactggc tgaaaattag tcagatgaat aatctggcgt 4200
   cagtcttctg gtcccttttc tgaagcctgg tattagcagt ccagctcatt taaatctctc 4260
   cttcactcta acttcaagct ctctctgcag aactgtacct gagcaccatg tgctacttag 4320
   ttgcctaaaa gaaaaactct tttgcacaga aaaattacat ttattaagga acacacctaa 4380
   atactatcat ttctgataaa agtgacagtt ggaatcattt gagaaccatg cagaattttt 4440
   ctggcagcag ttacccttaa caaatgtact gcgatgctgt gtgctggatg tagattccca 4500
   tggaaaaggg agtagctcca aaatcctaat agtaccatgt ggtctggcca tgaactaaag 4560
   cttttcaaac catatggaaa ctcattactt caaagcactt ccacagtcac cttattttat 4620
   tattattttc acattagcgc tatagcataa gggtaaagtg gaaaaatatt cccagcttat 4680
   agatggggag acgaacatgc agcaatcatt gttaatttcc ttatttctcc taatgtcacc 4740
   aatattctct tggaagttta cctgttcatt actttttaat agaatcgatt gagctattac 4800
   taggatttat tctttcccct ccatttctgg cattaacatt ctagaccaca atttcttccc 4860
   acatgcctag attacttcca taactttcta gatttctccc tacctatgtt ctcttctcct 4920
   tttaggttgt gttcagaatt gatatagtta taaaagtttg gctcagtaaa aaaaacacaa 4980
   gaatgttcca aagaaaggga acatcagcct ggagtgttcc tgaggaacta caaaatgttt 5040
   agcttccctg gagattgtca tagaaatgta aagggttgtg taagatgaga ccataagata 5100
   aagtgaggag caggttatat atcggctttt acattccatt tcaaggaatg cttatgctga 5160
   aagcaaggac attttaagag ctttaggtct gaaaattgca taatcaaatg tggatgctta 5220
   caaaatcaac cacactcatg ctgtaaaatg aagaaaataa attagtccat tgggagactg 5280
   tacagtttgg gatgggaggt aattaatttg gaagcctgtc agtaatcagg ccaagtgatt 5340
   atggtattct gtactaaagt gctggcaatg gggtgagtaa aggtaagttg ctttaaaaat 5400
   atagaggaaa tagacttaaa acacatgaga agtaacctat taaaaaatca aaaataattc 5460
   ataaactcta acttaagcac tggttttttt ttaatatttt tatttattta tgatagtcac 5520
   agagagagag agaggcagag acacaggcag agggagaagc aggctccatg ccaggagcct 5580
   gatgtgggat tcgatcccgg gtctccagga tcgtgccccg ggccaaaggc aggcgccaaa 5640
   ccgctgcacc acccagggat cccaagcact ggtttttaat ggtgattttt cctcaatagc 5700
   gaacaacaag agttaggttc taacttggaa atgatgagcc tggaatgatt gggtacatcc 5760
   agatggggat gtctccagga cagtttaatg tacatgactg agggtcaaga aagatataca 5820
   cttaggtgtc atctgtataa atggagcagc tgttgaatga ttacacatat ggttgtgtgt 5880
   tatatgcagg atgttaaaaa gggtgctgtt tattttatct atcttctcta cttggggcac 5940
   caatttgggg tcaaatatat aaaacgattc tttatccaaa gtgggtaagt ttaattttac 6000
   taaagcgatg aaaaaggtgg tataaagcct aaaaattaaa tatacatagg tacttatgat 6060
   gaacaagaca ctgttcgaaa tcacttgctt tttgtgccat gtaatccact tagaaactca 6120
   acagtagtcc ccactttacc tcaaagacat aaaataaagg accatcattt acctagaagt 6180
   ccaagaatgg agccaaatca tgtatacaat ttcttgaaac agcatgatta attcacattt 6240
   attaatgatt actctatgtc tagcatgaca ctagatgctg tgtactgaag tctctttttt 6300
   agtttatatt aggttacact acataacccc aaataatgaa tggcacagtg aaatgtaaac 6360
   ataaatacat atatgcacag gtgcccaaag gaatctacaa ctcattggca gctaagctca 6420
   gtgtatttca gtccctcttt tctgagaagc acatagaagg gaaagccagc cacctgctcc 6480
   cagtgcccct gaaccctgat ccagaaacag cctgacctgg tgggatatga aaagtttatt 6540
   ctctaatagt gaacaaggtt tcaaaagagc ttcggtgaac tcctgtgatt tcctatccac 6600
   atttcattct taaggtaatt ctatttgaat ggccttttca aaatttaagt tctatattct 6660
   catgctattt cctcatctgg tttgttcagt tggacactca aaggtcttgt tctgattccc 6720
   cttacctagt tacatttgat aaattggcat ttcgccctca cttcagctag gctgaattac 6780
   actttgcatt gctgatcttt ctggggttgg gggttcatgc atttctttag tctccaggct 6840
   gctcctttgc ttctctactc tgagatccat tccccactct ccctgctttg ctctgtcatg 6900
   gaagtccgaa tgccgcacac tgcaattctg gatggtgcta attagcaaat gggaagtgtt 6960
   agcagtacga ggaggaaggg acaatctagg gtattttttc ccttcacttg gatttgggcg 7020
   ccagcctctg gggtggccgt gtctccccca ggattctaga ttttactgga tgtgctgctg 7080
   actcctgacc cctgttcttt ctatctcctt taactctcct gcccaggggt gttactagcc 7140
   ctctgctttg ttaatctctg gtttgtttca tttgtcgtct gattggcttt tcagccacca 7200
   aaacttttat gcctattttg ctgtatgcaa tgtcctccat tgaactactt ggtatgaacc 7260
   atgttttctt gatcaatccc agattagtat actttttcat ctacttctag ctgaggatct 7320
   tccagctgaa tttgaactga ataaagacaa gataatgata gtccaaaaat aagtattttc 7380
   cctatgatcc agccttgact tcttttccat gacactctct ttggttttcg ttattcttta 7440
   tattcctctc ttcttgactc ttttcttagg ttttcctcac ttattaggat tgcttttgct 7500
   catttattac tcatttgttt gttcatttgt tgccatatcc catgctcttc cattattggg 7560
   gttatgactt ttttatacca tttctagcac acatttgcca agatttttca ggctgtctta 7620
   caaaaaaggt taaaatgcag actttgtata ttcatttttc catagtagat gtcaatttta 7680
   ttttgagttc aaatatattt aatgtggaaa atacaactct taacttccat agcattccct 7740
   acaaaattgt tagctacagc tgtcagcaaa tgctcaagaa ttcaagagtg gcttttagat 7800
   ctctgctaaa agggtggcct ttattaacct ccttggcttt tggcttttgt aaagaaaaga 7860
   tgtacttcca atgaatcttg attcgtcaca cttgcacaga gtcatacaca cgcacactac 7920
   aaaaatacag caaaacaatt ttttctactt ttccttttgc attattaaat cattgtctct 7980
   gcctggtgtt tatttaacca cttttggcat atctacatat tagttataga aacatgtaac 8040
   acatcctgcc ttttaaggga tgtttatctt ttcttctttc tttcccgata aataatgaac 8100
   tttttgaata ccttaatccc acttgagtgt ctgacaatcc ttgcataaaa tttgcataca 8160
   gtaaatgtgc aataattgtt ggttaaatag aaataaatat aaaaacactt aaatgtaaaa 8220
   tgtttaaaat cagaagtaac tccttctcat tttggaatac tgttacctat tctttttttt 8280
   aatccatctt cagtagataa tgctattagg gctctgaaag taacaaatac tttcataata 8340
   ttttgcttga aaaatgcatg acccagattc cataagatta tgatggaaat atcttccagt     8400
   gcaaaacttg acgcatggca ggagatcaac aagtgaatgc tggttgaaaa aatgaattgc     8460
   cagtccctga gactgtctca tttgttttca taacttctac tttattcata tttttatccc     8520
   tttgaactct tagcaccaag tattttctca gtatgctttt acaaattgaa tgaatgagat     8580
   aactttcaca cgttgattct cacctacttt gattgctttg gagtctaaac caagtttcac     8640
   tggtggaaga atggaaaata ttataccatt acctaggaag tgttatgtgg atcctatctt     8700
   tgagtcatga attttacagc tggactttta tcatgtcatt agtagatggc tcatgatcta     8760
   attcctcctt ggagcacagg tagaggccca gtacactcca cagttatata cctcctatat     8820
   atagtttatt gtgagtattt gaaggcagaa atgtcatcct gttcatcttt gtgctttaaa     8880
   ggttcaagtc ccattacatt gcaccactgc tactctggcc ctctgaacat ttataagagt     8940
   cataggtata tagttgatat ataataaatg tttactaaat gaataattta ataaaataat     9000
   tgactcatgc aattgccctg aaaagcaggg gaatgaagca gccagcagta cttccaggaa     9060
   gcaactgaga aacacccttt ggcacccacc attcaatcaa cttaacatca gccttgaagc     9120
   ttaagaaata aaattaaggg ccaagtaagc atagctcaag ccaactactt ttgcattccc     9180
   caggccactg attgcctcac taaatctctc actttgctga atatgtgaaa tcaattatta     9240
   ttataggata caaaagacag aaactttttg gaaaattgtt ttgattatta ataaaagatg     9300
   acaatgtcaa ggcatgagtt accaccataa aactccacgt tagcattatt tgaattcagt     9360
   agaacaaaga caagtagatt tgtcaaatta caaccctgat tgtgtctcct atgcatctcc     9420
   attacccaaa ccagtgtaat gttctcaata gttgctttct tccaataggg gagcactgta     9480
   aggtcttata aaaataccta actttcaggt tcccacagaa actgagggaa tcaccccaga     9540
   tctaacctcc cactcacctt tgtcaacttc actgccattt tatttctttg atttctgccc     9600
   agtttcttgg cctctaactt cggtcctcca gaccagaaac ctttgaagca ctgtgcagac     9660
   tatctgttct tgactctgtg ccaaccttgg atattgcctt aaagatttgc ctgacttgat     9720
   ttccagcttg agaattagca tagctctact tttatcgtgg tgcccatctc tggtccttta     9780
   gaagctcaaa cttcaaccaa caaaggccca ggaccctttt gcttccccaa gaggaatgaa     9840
   tgttcaaaca gaatcatctc cttctgtaaa cagtgatctg tgtccttttc ctcaccctct     9900
   ttccccattt aaaacctctt tgtttgcata cttcatttgt ccagttctta ctgacagttc     9960
   cagactatct cagccatcaa ctcaggaagc tatactaacc aatttgtaac tgaacatctc    10020
   aagcatacct tgatatgcct ctcattttcc ccttatctcc tcctgcctca tatttgaata    10080
   atcttttaag gtataaatat gttatattaa ctctgttaat gactgtgatt atttttgaga    10140
   gctctagggg agctatatta tataattctt ggtatctctc actgtacttg cagagtgctt 10200
   tgcacacaag taaaccacag aatggtatac aaatgaatat ttaaatgttt agctgttttt 10260
   gaccaattat tcttaatatt ggcttcttat gacttattac cttgtgaaat attttgttag 10320
   ttgcatatct agaaaggttg ataaattcag atcagctcca ccattcttgt gtaattatta 10380
   ttattttcta aatgttcaat aatttaaata gtacttatgt acaattaatt cttgatttga 10440
   tctatggcat aggtctgtgc ccaggtgctt gggaaattga ctttgacatg gaggtttgga 10500
   tgcaggcggc taatagggaa ataaactagg caatagcacc agtgctagtg tgaaaaacaa 10560
   aggattcgac agaaaaagtt gaactccaat gcaagtgaaa atgaggcctt ggggcacctg 10620
   ggtggctcag gttgtgattc tggggtcctg ggatcgagtc ctacaacaga ctccctgcag 10680
   ggagcctgcc tctccctctg cttatgtctc tgcttctcct tctgtgtctc tcatgaataa 10740
   ataaataaaa tcttaaaata aataaaataa aaaatggggg ggggagggaa tggagcagcc 10800
   ataaggacta gttatatgag tgttggtttt ctcaaaagca tacttggaga aaatgttaac 10860
   tttttaatga tagatttaat atattaatat atgccaagta cttagtgtaa tgcttatcat 10920
   gtggtcactg ctcgctgaat tgaagttgcc tgaatctagc ttggaggcag gtgtgagtaa 10980
   gtcataaatt aactatgaaa tgacctttat ggctttagca actctataag atcccatagt 11040
   gaccacattc attattaagt aatctggtct taatcttgat aactgatttt gaatttatga 11100
   ctcctctttt actccctcat ctacatctct tcaactgcat tgttaatctt ttcagtcctg 11160
   tgcgtgtcat atcaacttcc tcccgttacc ttgtctcatg atgttctact ctattttctt 11220
   ccagaaagtg aactgtccct cctctctata aaatcacatc ttaaatgtcc tctctcccac 11280
   ttacataagg cttccttatg caaggattac tttgtttttt agacttcttt acctcctccg 11340
   aagtcttgca cttattaata tgtattactc ttttataaat caggattttg gagattaatt 11400
   taaatacagt aaaattcacc ttttgagttt gccaaatggc aaactgtcat tcaaccaaac 11460
   taccattaag ttgtagaaca tttcaactac catgaaaagt ttcctcataa ctatttgtgg 11520
   tcagtcccat cctcctccac cccccatacc tggtgaccac tagcggagtt tctgtcttca 11580
   cagttttgcc tttcccagaa tttcatttaa acagagtcat ataatatgtg ttttctggca 11640
   taatgctttt gaggtccatt cattgtttca tgtatcaata ggtcattcct tctaatggct 11700
   aagtagtatt ccattacatg gatataccac aatttgttta tatatttact acgttgatgg 11760
   gcctctggct tgatttcaat atttaactat tatgaataag tttatgtaaa tattaatata 11820
   cagatctttg tgtggaaaat gtttcaattt tttcccatta ataatgctga gggaaactgc 11880
   tggattatga ggtataattg tttaacatta cttgaaaatt ccaacttctt tttgtacata 11940
   tttgtactgt tttgagttat aaaaaactgt gtttctgttt aatcacaaac taattaaact 12000
   gggtaaatat ttttttaact ttagatacct acttgctata tagtaacacg tcggtgtggt 12060
   cttaattcgg attttactaa tgactaatga tgttgagcat tgcttccagc cagctatgta 12120
   tctcctttgg tggagtgtct gtgtaaatct tttgcccatt gaaaaaactg ggttattttt 12180
   tcttcttctt gattcctatg agtattcata tgttctaaat atgcctctat caaacaggta 12240
   ttttgaccat attttctctt catctgtagc ttgtctttac attctctaac agtccctttg 12300
   tgaagcagat tttatttatt tatttattta tttatttatt tatttattta ttattttttt 12360
   aaattttcat gagagacaca cagagggaga ggcagagaca ggcagaggga gagaagcagg 12420
   cttcatgcca ggagcctgat gtgggcctca atcccgggtc tctgggatca tgccctgggc 12480
   cgaaggcagg cgctaaactg ctgagctacc cagggatccc cctgtgaagc agattttaat 12540
   gttgattaag tccagttatt tttttctagg tttcctttgt gtcctattta aaatatcttt 12600
   gtctaatcaa aagtcacaaa gttgacaaag tcctcctgtt ttttctcctg gaagttgtac 12660
   agttttaatt tttatatttt taagtctgtg atacatttaa tttcttctat aaggtgtaag 12720
   gtaagggctg aggctcatat tttgcatgta gatgtgccat tgtttcagca ctctttgctg 12780
   aaaagactac ttttcccttc aaattacctg ggaagttttt tgtaaatcaa cagaatatat 12840
   acgtatggat gtataccttt cattatggat tagtgaaaag atcatgattt tggggttata 12900
   ctaacattta aatcctgtct ctatcatttt ctatgttttc taaggccatt tattatgaat 12960
   cagttcctta atctgaaaaa ataattaaaa tataaaattt ttgaggttta ataactgtaa 13020
   aataagcaag acatattaaa tttttagtgt ccaggcagta ctcaaaagtg gtatttgtgt 13080
   ttacttgttt ttttttgttg ttgttgtttt tttttttttt acttgttttt aacaattttc 13140
   aaatcttccc tttctagtta gatgagtagt ctcttaagtc agggactgtt ttgttttgtt 13200
   ttgttttttt ctaattctac aattacagcc acaatcttca gggtagatat tgtgtcctat 13260
   ggaggataag atactgaggg tggtttgaat tttcaaagaa gatgatgaag accatgacca 13320
   ctggctgtgt aagcaaatta ttaaattaat caaagttcag aggagatcaa gaatgttggg 13380
   ctatctctat gctccttaat catgtacata ctgctttgtt ttatcctctc tctgttcaat 13440
   aggacatgaa attcaacata aatcctactg atttagacca aagatcaata gcattttatt 13500
   cactataaat tgcttcatta ttttgcaggg tctccattct cataacctaa aattaaccgt 13560
   ttctttaaaa ttgcccaaat tccaaagtat aagccaaaca aaattgttga ttgctttggg 13620
   aagggtttta tgaagttaag aggagcagaa atacattgct ttcaaggaat ttgttattta 13680
   aaatgaatgc aaaaatgttt gcagaataga ttgttgaact gggagtcaaa ttagtttggt 13740
   tcaaattcca ctctgccttt gaataaaaag caagttgttg aactgcctta actgccaact 13800
   ttattatcca taaataggag ctaattcacc tcaaaaggcc tttgcatgca cgaatgagat 13860
   aataaatgtg aaagcacttt gtaatcacta acatcctata caaataaaag tcattgttat 13920
   tacgatgatt caaagccaac tctagaaaga atatgttatt tgttgatcaa tgaaaatgac 13980
   ccctggagac ctccttcccc tagtcaagga acactctact aagatgtaat actacctgtc 14040
   atttcaccat gtgcactgca ttgctttatg ttaagtaagc cctctcagat ttccttttaa 14100
   aatggcaata cctcctaaag agtaataatt aaagtcacag ctcagagtgc ataggtctca 14160
   aggactttct gttaaatacc tggcacctga gcaactttta catgtgtctt ttcagcagct 14220
   ctgactaggg cagcaaaaca taaaaacatg agtcctagaa gacttgaaaa gagtaaatgg 14280
   ggaaaaaaaa ccaacctaga aactagagaa tatgttagag agaggtagaa aagaacaaga 14340
   gtctctttcc aattgtccag ggtaagcctt gtaaatagca gacattaaag atatattgca 14400
   ctgataatta taaccagtat gtatttttta aattactgag gtataattga cataaataat 14460
   tgcttgtatt taagtggaca atgtgatgaa ttggggccta tgtagatgcc catgaaacca 14520
   ttaccacagt caagaaaatg aacaaaaccc tcaaccctct gcctactgca aatgtcatcc 14580
   ctctggttcc tcccattttc ccttccatgg cctacgtcca tcattccgta gtactttgaa 14640
   tttcttagca ttttatgaaa atagaatcat atgatatata ttctttaaaa aatctggttc 14700
   ctttttaaaa aagattttat ttatttgaga gagagaaaga gagtgcacaa gtggagagag 14760
   gagccaaggg agagggagaa gcagactccc cactgagcag gaagcttgat gggagggctc 14820
   aatcccagga cctggagacc atgacctgag ctgaaggcag acatttaacc aactgagcca 14880
   cccaggcacc caaatctggt tcctttcact ctgcacagtt attttgaggt tgatccaggt 14940
   tatactatgt atgaagttaa ttccttttta ttgctgaata gtattccatt gtatgactat 15000
   cagtttgttc ttctacatgt tgatggatgt ttggatgatg ccattgttta acttttatga 15060
   ataaagcttc tatgaacaca agcatacaac tctttctata gacacttgct ttcatttatc 15120
   aggtaactat ccaggagcaa gatagctgaa ttatatgtca agtatatttt taattttgta 15180
   agaactgttt tccaaagtgg ttacaccatt ttgcttgacc accagcagtt cattggagtt 15240
   ccaattcctc catatttctc caatacttgg tatggccagt gtttttttgt cttaaccatt 15300
   ctaataggca tgtagggata tcactatggt ttgatttaca tttcattaat gactaataat 15360
   atttagcatt atttgatgtg cttatttgcc aactatgtaa atcttctgtg gtgaaatgac 15420
   tttaaatttt tacctttttg ttggtttgtt ttatcttcac atcattggac tttgaaaatt 15480
   ccttattttg agtacaagtc tattatcagt taaatgcttt tcaaatattt tccccagtct 15540
   gtgacgtatc ttttccttct ctttgtagac tgttgaatct atttctggac tctattctgt 15600
   tcattgacct atttctctat ctttacacaa ataccacatt tttattacta tagccttaaa 15660
   ataagtctag aaatcggtag tatcagtcct ccaacatatt cctttctttc aaagtgagta 15720
   tggctatcct agattatctt tccctaagtg ttttaaatct gtttgttaat tcaacaacag 15780
   gaggaggagg aagagaattc tggtggagtt ttggttgtcg tttctttgaa tatttagatc 15840
   aatttggggg agaagtgaca taatgttgaa tcttccaatc taactatttc tgagcaataa 15900
   gtaaggcagt atgctatcgt ttagttttct tgtttcttgt gcttgaggtt cattgagttt 15960
   cttatatctg tgagttaata atttccatca aatttggaaa atttgcaggc attattcctt 16020
   caaatacatt ttctcctctc tccttcaagg gatccagttt tatgtatatt aaaccatttg 16080
   aaattgtccc actacttgct g 16101
<210> 2
   <211> 20
   <212> DNA
   <213> Artificial
<220>
   <223> Primer
<400> 2
   ccttcccttg ggagattagc 20
<210> 3
   <211> 20
   <212> DNA
   <213> Artificial
<220>
   <223> Primer
<400> 3
   aatgatgtgt ttgggggaaa 20
<210> 4
   <211> 20
   <212> DNA
   <213> Artificial
<220>
   <223> Primer
<400> 4
   ggtgacgtct ggattggatt 20
<210> 5
   <211> 20
   <212> DNA
   <213> Artificial
<220>
   <223> Primer
<400> 5
   acttccccac tttgcctacc 20
<210> 6
   <211> 20
   <212> DNA
   <213> Artificial
<220>
   <223> Primer
<400> 6
   caaatgtgct gatggaaacg 20
<210> 7
   <211> 20
   <212> DNA
   <213> Artificial
<220>
   <223> Primer
<400> 7
   aactgcagcc acatgtgaag 20
<210> 8
   <211> 20
   <212> DNA
   <213> Artificial
<220>
   <223> Primer
<400> 8
   gcagggcatt tttgaggtag 20
<210> 9
   <211> 20
   <212> DNA
   <213> Artificial
<220>
   <223> Primer
<400> 9
   gaatagccct gtggggtgta 20
<210> 10
   <211> 20
   <212> DNA
   <213> Artificial
<220>
   <223> Primer
<400> 10
   tcactcctgc tcacatctgg 20
<210> 11
   <211> 20
   <212> DNA
   <213> Artificial
<220>
   <223> Primer
<400> 11
   tctcgacaga caccgtttca 20
<210> 12
   <211> 20
   <212> DNA
   <213> Artificial
<220>
   <223> Primer
<400> 12
   aacggaagca ctgaatcaaa 20
<210> 13
   <211> 20
   <212> DNA
   <213> Artificial
<220>
   <223> Primer
<400> 13
   gcaaatccaa gccctattca 20
<210> 14
   <211> 25
   <212> DNA
   <213> Artificial
<220>
   <223> Primer
<400> 14
   gctcagagtg cataggtctc aagga 25
<210> 15
   <211> 26
   <212> DNA
   <213> Artificial
<220>
   <223> Primer
<400> 15
   ttttggggtt ttgttgctat tgttgt 26
<210> 16
   <211> 26
   <212> DNA
   <213> Artificial
<220>
   <223> Primer
<400> 16
   tgctgtgtta aaaagctatc cttgga 26
<210> 17
   <211> 30
   <212> DNA
   <213> Artificial
<220>
   <223> Primer
<400> 17
   ttcatttaca atgcctaggg atttttagga 30
<210> 18
   <211> 24
   <212> DNA
   <213> Artificial
<220>
   <223> Primer
<400> 18
   tcttctgagt tctcttccca caca 24
<210> 19
   <211> 22
   <212> DNA
   <213> Artificial
<220>
   <223> Primer
<400> 19
   gaggcaactg gagttcctat gg 22
<210> 20
   <211> 20
   <212> DNA
   <213> Artificial
<220>
   <223> Primer
<400> 20
   tgcagggtct ccattctcat 20
<210> 21
   <211> 20
   <212> DNA
   <213> Artificial
<220>
   <223> Primer
<400> 21
   gcttaccctg gacaattgga 20
<210> 22
   <211> 20
   <212> DNA
   <213> Artificial
<220>
   <223> Primer
<400> 22
   tgcagggtct ccattctcat 20
<210> 23
   <211> 20
   <212> DNA
   <213> Artificial
<220>
   <223> Primer
<400> 23
   gcttaccctg gacaattgga 20
<210> 24
   <211> 7
   <212> DNA
   <213> Canis familiaris
<400> 24
   tgaggag 7